(19) Europäisches Patentamt / European Patent Office / Office européen des brevets

(11) **EP 3 592 434 B1**

(12) **FASCICULE DE BREVET EUROPEEN**

(45) Date de publication et mention de la délivrance du brevet:
**24.01.2024   Bulletin 2024/04**

(21) Numéro de dépôt: **18712957.2**

(22) Date de dépôt: **06.03.2018**

(51) Classification Internationale des Brevets (IPC):
**A61K 8/73** *(2006.01)*      **A61K 8/97** *(2017.01)*
**A61Q 7/00** *(2006.01)*      **A61K 36/88** *(2006.01)*

(52) Classification Coopérative des Brevets (CPC):
**A61Q 7/00; A61K 8/73; A61K 8/97; A61K 36/88**

(86) Numéro de dépôt international:
**PCT/FR2018/050506**

(87) Numéro de publication internationale:
**WO 2018/162834 (13.09.2018 Gazette 2018/37)**

(54) **UTILISATION COSMÉTIQUE D'UN EXTRAIT D'AGAVE TEQUILANA POUR AMÉLIORER LA CROISSANCE DU CHEVEU**

KOSMETISCHE VERWENDUNG EINES EXTRAKTS VON AGAVE TEQUILANA ZUR VERBESSERUNG DES HAARWACHSTUMS

COSMETIC USE OF AN EXTRACT OF AGAVE TEQUILANA FOR IMPROVING HAIR GROWTH

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **07.03.2017   FR 1751832**

(43) Date de publication de la demande:
**15.01.2020   Bulletin 2020/03**

(73) Titulaire: **Laboratoires De Biologie Vegetale Yves Rocher**
**56200 La Gacilly (FR)**

(72) Inventeurs:
• **LAPERDRIX, Céline**
**78470 Saint-Remy-lès-Chevreuse (FR)**
• **LUBRANO, Christian**
**92310 Sèvres (FR)**
• **ZOZINE, Sophie**
**78330 Fontenay-le-Fleury (FR)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
EP-A1- 1 541 117      BR-A2-102012 012 704
CN-A- 106 420 461      ES-A6- 2 009 151
FR-A1- 2 551 972      JP-A- 2000 136 142

**Description**

**Domaine technique**

La présente invention se rapporte à une utilisation cosmétique non thérapeutique d'un extrait d'Agave tequilana pour une amélioration de la croissance du cheveu.

La présente invention trouve une application dans le domaine de la cosmétique et de la dermatologie, plus particulièrement de la cosmétique capillaire.

Dans la description ci-dessous, les références entre crochets ([]) renvoient à la liste des références présentée à la fin du texte.

**Etat de la technique**

La peau est un organe vital à part entière qui se compose de trois tissus distincts, assumant chacun différents rôles grâce à différents types cellulaires et différentes structures.

Le tissu le plus en surface et donc le plus exposé est l'épiderme. Cet épithélium pluristratifié (Malpighien) et kératinisé, dont la partie la plus externe est la couche cornée, se compose de différentes cellules associées à de nombreuses fonctions de barrière et de protection. Les cellules majoritaires sont les kératinocytes qui, par leurs processus de prolifération/différenciation, aboutissent à la formation de la couche cornée connue pour ses propriétés hydrophobes, son aspect compacte et étanche. Le rôle majeur de l'épiderme est d'apporter à la peau, et donc au corps humain, une première ligne de protection contre les agressions extérieures, comme les agressions physiques, chimiques, hydriques et bactériologiques, notamment via la différenciation et le stratum corneum.

En position intermédiaire, le derme est aussi un tissu conjonctif investi majoritairement de fibroblastes et de protéines matricielles donnant à la peau ses qualités de compressibilité et d'élasticité connues. Au sein de la trame conjonctive, s'intercalent aussi d'autres cellules et structures, tel un important réseau circulatoire et nutritif, constitué des vaisseaux sanguins et des capillaires lymphatiques, ainsi que les annexes épidermiques : cheveux, poils, ongles, glandes pilosébacées et glandes sudoripares, qui prennent naissance dans le derme profond.

L'hypoderme, situé en profondeur et constitué en majeure partie de lobules graisseux (adipocytes), assure une fonction de support primaire, de protection mécanique et thermique et joue aussi un rôle de stockage des réserves énergétiques rapidement mobilisables pour tous les besoins biologiques, comme par exemple le renouvellement cellulaire, la défense de l'organisme ou la contraction musculaire.

Le cuir chevelu est la partie de la peau située sur le crâne qui développe une pilosité particulière : les cheveux. Parmi les annexes de la peau, les follicules pileux présentent la particularité d'être une invagination tubulaire de l'épiderme qui s'enfonce dans le derme voire l'hypoderme. Cette invagination épidermique se renfle à son extrémité interne pour former un bulbe composé de cellules épithéliales, les kératinocytes qui vont proliférer et se kératiniser pour former le cheveu. Le cycle de vie du cheveu comprend trois phases : la phase de croissance dite anagène (dure 3 à 5 ans), la phase de dégradation jusqu'à l'expulsion dite catagène (dure 15 à 20 jours) puis la phase de repos dite télogène (dure 2 à 3 mois). La croissance du cheveu est possible grâce aux interactions entre les kératinocytes, les fibroblastes de la papille dermique et les cellules endothéliales des vaisseaux présents dans cette zone. En effet, lors de la phase anagène, des signaux sont envoyés par les fibroblastes de la papille dermique pour stimuler le processus d'angiogenèse qui va permettre d'alimenter la naissance puis la croissance du cheveu. La migration des cellules endothéliales vers le bulbe du follicule pileux et la formation des vaisseaux jouent donc des rôles prépondérants dans cette phase de croissance.

De par leur position externe et leur longue durée de vie, les cheveux accumulent les dégâts engendrés par les stress environnementaux comme les UVs, la pollution, le froid, mais également par les traitements esthétiques (séchage, frisage, lissage, coloration ...) ou l'utilisation de produits agressifs (shampoing trop récurrents ou mal adaptés). De plus, ils sont affectés par des facteurs internes comme le stress, les modifications hormonales ou encore le type d'alimentation.

Actuellement il existe peu de produits cosmétiques stimulant la croissance du cheveu, il s'agit surtout de traitements hormonaux, de compléments alimentaires ou de dérivés d'acides aminés (taurine ou arginine) issus de processus de biotechnologie non naturels.

Le document CN 106 420 461 (Canton Damekiss Daily chemicl Factory) décrit une composition comprenant les plantes suivantes: osmanthus, lespedeza, gerbera, rhodiola rosea, agave et rose. Cette composition peut se présenter sous forme de shampooing permettant de nourrir les cheveux et le cuir chevelu, hydrater les cheveux et le cuir chevelu, réparer le cuir chevelu et maintenir la santé de celui-ci.

Le document JP 2000 136142 (Ichimaru Pharcos Inc.) décrit l'utilisation d'extraits de sisal, d'agave et de forsythia pour leur action anti-allergique, passant par un effet inhibiteur de la libération d'histamine (cf. [0012]). L'action des préparations décrites dans ce document sur la peau et le cuir chevelu est une amélioration des maladies inflammatoires cutanées allergiques (par exemple les rougeurs, les oedèmes, l'eczéma), la dermatite atopique, et la peau rugueuse et sèche due à ces réactions inflammatoires (cf. [0012]).

Le document BR 1020 1201 2704 (De Campos Catarina Ledi) décrit l'utilisation d'une combinaison de plantes comprenant l'aloe vera, le piment, l'ail, le romarin, le jaborandi, l'acérola, l'avenca, l'agave et la bardane pour un traitement progressif du cuir chevelu dans la lutte contre les pellicules, la séborrhée, le psoriasis, la chute des cheveux ou la calvitie. Les effets de l'agave qui sont décrits dans ce document sont les effets émollients et antiseptiques.

Le document ES 2 009 151 (Llaurado Grau José Maria ; Lopez Valverde Juan Antonio) décrit une composition consistant en un mélange de 1 à 5% de jaune d'oeuf, de 1 à 4% d'acide citrique, de 1 à 4% d'acide salicylique en poudre, de 0,5 à 10% d'huile végétale, de 1 à 5% de liq. bio-soufre, 5-10% de polyvinylpyrrolidone iodée, 30-50% d'extrait d'agave americana, 1-4% de teinture d'écorce de savon, 1-4% de goudron de houille, 10-20% d'excipient anionique et 10-20% d'excipient d'amidon.

Le document FR 2 551 972 (Boudiz Moussaad) décrit un produit arrêtant la chute des cheveux, éliminant les pellicules et favorisant la repousse des cheveux, composé de 10 plantes : romarin, fenugrec, tan, genévrier, clou de girofle, grains de cressonnettes, pétrole brut et agave.

Le document EP1541117 décrit l'utilisation cosmétique non thérapeutique de fructanes d'Agave tequilana comprenant des inulines naturelles pour protéger la fibre capillaire en tant que conditionneur, pour améliorer l'aspect et le toucher de la fibre capillaire.

Il reste un réel besoin de trouver de nouvelles compositions et/ou composés d'origine végétale et/ou naturels permettant d'assurer et/ou d'améliorer de façon effective la croissance, la brillance et la douceur des cheveux.

**Description de l'invention**

La présente invention a précisément pour but de répondre à ces besoins et inconvénients de l'art antérieur.

Les inventeurs sont les tout premiers à utiliser des extraits d'agave permettant précisément de répondre efficacement aux besoins précités.

L'agave est une plante monocotylédone, actuellement classée dans la famille des Agavaceae. Les espèces les plus courantes sont *Agave tequilana, Agave americana* et *Agave attenuata.* L'agave est endémique sur le continent américain et est présente du sud du Canada jusqu'au Nord de l'Amérique du Sud et les îles Caraïbes, en particulier au Mexique et dans toute l'Amérique centrale (Maria de la Soledad ALONSO GUTIÉRREZ, thèse, « Valorisation de la bagasse de l'Agave tequilana W. cv azul : caractérisation, étude de la digestibilité et de la fermentation des sucres », 2005 ([1])).

Les agaves présentent une partie souterraine ayant la forme d'un rhizome, et d'une partie aérienne présentant de longues tiges et de grandes feuilles fibreuses, disposées en forme de rosette et qui finissent en épine. Les fleurs se trouvent à la fin de la tige et sont de couleur jaune-vert ([1]). L'inuline est un polysaccharide de réserve trouvé dans les plantes de la famille des Artéracées, notamment dans les bulbes et racines de dahlias, de topinambour, d'artichauts, de pissenlits et dans la chicorée, cette dernière étant la source la plus utilisée industriellement. Constituée de 30 à 40 unités de fructose liés par des liaisons glycosidiques en $\beta$ (1-2), et d'une à deux molécules de glucose placées en bout de chaîne, l'inuline est en réalité un glucofructosane. Les inulines d'agaves sont des réserves glucidiques de type fructanes (également appelées fructosanes). Des polyfructosanes ont été extraits de l'Agave *tequilana Vlleber var. azul,* âgée de 8 ans. La caractérisation de ces fructosanes a donné comme résultat un degré de polymérisation compris entre 3 et 29, et une composition complexe de fructo-oligosacharides avec des liaisons de $\beta$ (1-2) et $\beta$ (2-6) différentes des inulines rencontrées dans les autres plantes (López, M.G. ; Mancilla, N.A. ; Mendoza-Díaz, G. (2003). : Molecular structures of fructans from Agave tequilana Vlleber var. azul. J. Agric. Chem. 51, 7835-7840 **([2])** ; **([1]))**. Les inuline de chicorée ont une structure différente de celle des inulines d'agave. Les inulines de chicorée présentent en effet des chaines linéaires de fructoses liées par des liaisons beta 2-1 alors que les inulines d'agave, même si elles sont composées également principalement de fructose, ont des structres latérales branchées à la structure principale par des liaisons bêta 2-6 (López, M.G. et al. **([2]))**.

Les inventeurs sont les tous premiers à avoir découvert que des extraits d'Agave *tequilana* possèdent de manière inattendue un effet d'amélioration de la croissance et de l'aspect du cheveu et du cuir chevelu.

En effet, les inventeurs ont mis en évidence un effet des extraits d'Agave, notamment d'Agave *tequilana,* sur la formation des vaisseaux sanguins et la migration des cellules endothéliales, pour un effet de stimulation de la croissance du follicule pileux.

De plus, les inventeurs ont mis en évidence un effet des extraits d'Agave sur la stimulation du renouvellement des kératinocytes épidermiques, pour un effet sur la brillance et l'aspect du cheveu et du cuir chevelu.

Ainsi, l'objet de l'invention se rapporte à l'utilisation cosmétique non thérapeutique d'un extrait d'Agave *tequilana* pour une amélioration de la croissance du cheveu dans laquelle ledit extrait d'agave comprend des fructo-oligosaccharides ayant un degré de polymérisation inférieur ou égal à 10 et/ou des inulines ayant un degré de polymérisation compris de 10 à 60.

On entend par « amélioration de la croissance du cheveu », toute amélioration, qualitative et/ou quantitative, de la vitalité et/ou de la pousse du cheveu, en longueur et/ou en diamètre. Ces améliorations peuvent être mesurées par toute méthode de mesure de la pousse, de la densité des cheveux ou de leur répartition des phases connue de l'homme du métier, par exemple au moyen d'un trichogramme comprenant le prélèvement d'un coup sec une quarantaine de cheveux sur 3 zones spécifiques du cuir chevelu pour étudier les racines et le diamètre des cheveux au microscope ou sur lecteur de microfiche, ou d'une macrophotographie avec rasage d'une petite zone qui est ensuite photographiée grâce à une caméra vidéo, pour comptabilisation exacte du nombre de cheveux et suivi avec un logiciel spécialisé, ou d'un phototrichogramme comprenant l'utilisation d'un logiciel spécialisé (Tricoscan™) pour comptabiliser le nombre de cheveux total, après réalisation d'une macrophotographie avec rasage.

On entend par « amélioration de l'aspect du cheveu » toute amélioration, qualitative et/ou quantitative, des propriétés optiques de réflexion et/ou tactiles de douceur et/ou de la régularité physique du cheveu en général. Ces améliorations peuvent se mesurer par toute méthode de mesure connue de l'homme du métier, par exemple par mesures de réflectance ou detribologie acoustique.

On entend par « amélioration de l'aspect du cuir chevelu » une stimulation, qualitative et/ou quatitative, du renouvellement de l'épiderme du cuir chevelu et/ou une amélioration de l'homogénéité et la régularité du cuir chevelu. Ces améliorations peuvent se mesurer par toute méthode de mesure connue de l'homme du métier, par exemple par photographies standardisées réalisées sur un appareil stéréotaxique permettant grâce à une distance fixe et à un éclairage standardisé des photographies globales du cuir chevelu reproductibles avant ou après traitement.

L'utilisation cosmétique décrite peut permettre de faire briller le cheveu et/ou adoucir le cheveu et/ou améliorer l'éclat du cheveu.

Avantageusement, l'extrait d'Agave peut stimuler le renouvellement des kératinocytes, et/ou la formation des microtubules et/ou la migration des cellules endothéliales. Sans vouloir être lié par l'explication d'un mécanisme d'action, il semble que la stimulation du renouvellement des kératinocytes et/ou la formation des microtubules et/ou la migration des cellules endothéliales soit à l'origine des effets cités précédemment.

On entend par « extrait d'Agave » au sens de la présente invention, tout extrait d'Agave *tequilana* permettant d'obtenir au moins l'un des effets cités précédemment.

L'extrait d'Agave *tequilana,* comprend des fructanes. Les fructanes comprennent des fructo-oligosaccharides ayant un degré de polymérisation inférieur ou égal à 10 et/ou des inulines ayant un degré de polymérisation compris de 10 à 60.

Ces fructanes sont donc issus directement de la plante, et sont donc naturels, c'est-à-dire non modifiés chimiquement, notamment ne comportant pas de groupement à caractère hydrophobe greffé chimiquement.

Les fructo-oligosaccharides peuvent avoir par exemple un degré de polymérisation compris de 3 à 10.

Les inulines peuvent avoir un degré de polymérisation par exemple compris de 10 à 29.

L'extrait d'Agave peut comprendre ou être constitué uniquement de fructo-oligosaccharides ayant un degré de polymérisation inférieur ou égal à 10. Alternativement, l'extrait d'Agave peut comprendre ou être constitué uniquement d'inulines ayant un degré de polymérisation compris de 10 à 60. Par exemple, le ratio fructo-oligosaccharides sur inulines peut être compris de 100/0 à 0/100, les bornes 0/100 et 100/0 n'étant éventuellement pas incluses. Le ratio peut être par exemple de 75/25, ou de 25/75, ou de 50/50.

Avantageusement, un extrait d'inulines ayant un degré de polymérisation supérieur à 10 peut en outre présenter un effet de protection de la fibre capillaire par amélioration de l'hydrophobicité de la fibre capillaire. La Demanderesse a en effet identifié que de telles inulines possèdent une action hydrophobe permettant une protection de la fibre capillaire, et ce malgré leur caractère hydrophile. L'utilisation de ces inulines permet ainsi d'améliorer le caractère hydrophobe du cheveu, notamment par amélioration de l'hydrophobicité de la fibre capillaire.

L'utilisation d'un extrait d'Agave comprenant un ratio fructooligosaccharides sur inulines de 50/50 permettrait ainsi de combiner l'effet d'amélioration de la croissance du cheveu et celui de protection de la fibre capillaire par amélioration de l'hydrophobicité de la fibre capillaire.

L'extrait d'Agave peut être obtenu par extraction des carbohydrates à partir de la bagasse, clarification de la liqueur brute obtenue à l'issue de l'extraction puis séparation pour obtenir une fraction de fructanes, notamment de fructo-oligosaccharides ayant un degré de polymérisation inférieur ou égal à 10, et/ou une fraction d'inulines ayant un degré de polymérisation compris de 10 à 60.

Un extrait d'Agave *tequilana* peut être un extrait commercial, par exemple le produit Metlos® (NEKUTLI), caractérisé par des fructooligosaccharides ayant un degré de polymérisation inférieur ou égal à 10, ou le produit Metlin® (NEKUTLI) caractérisé par des inulines vraies ayant un degré de polymérisation supérieur à 10. Il peut s'agir d'un mélange des produits Metlos® et Metlin®, dont le ratio peut être égal par exemple à 75/25, ou à 25/75, ou à 50/50.

Une composition cosmétique ou dermatologique, améliorant notamment la croissance du cheveu, comprenant des extraits d'Agave *tequilana* et un véhicule cosmétiquement et/ou dermatologiquement acceptable, ladite composition comprenant de 0,05 à 5 % en poids d'extraits d'Agave par rapport au poids total de la composition, ledit extrait d'agave comprenant des fructooligosaccharides ayant un degré de polymérisation inférieur ou égal à 10 et/ou des inulines ayant un degré de polymérisation compris de 10 à 60 est décrite. Toutes les caractéristiques d'extraits d'Agave mentionnées ci-avant pour l'utilisation de l'invention, ainsi que toutes les caractéristiques de l'utilisation d'un extrait décrites ci-avant, s'appliquent mutatis mutandis à la composition décrite.

On entend par « composition cosmétique », toute composition à visée cosmétique, c'est à dire esthétique, une composition pouvant être mise en contact avec les parties superficielles du corps humain, par exemple l'épiderme, les systèmes pileux et capillaires. Avantageusement, une composition cosmétique permet, exclusivement ou principalement de les protéger, parfumer, maintenir en bon état, modifier leur aspect ou en corriger les défauts esthétiques superficiels.

Dans la présente, on entend par « composition dermatologique » toute composition à visée dermatologique, c'est à dire une composition pouvant être mise en contact avec les parties superficielles du corps humain, pour un traitement de la peau, notamment du cuir chevelu, et des phanères, comme les cheveux ou les poils.

Par « véhicule cosmétiquement ou dermatologiquement acceptable », on entend un véhicule adapté pour une utilisation en contact avec des cellules humaines et animales cutanées, en particulier les cellules de l'épiderme, sans toxicité, irritation, réponse allergique indue et similaire, et proportionné à un rapport avantage/risque raisonnable.

Le véhicule cosmétiquement acceptable peut par exemple être choisi parmi l'eau, la glycérine, gel d'aloe vera, un extrait de plante aqueux, les tensio-actifs d'origine naturel.

Un procédé de préparation d'une composition cosmétique ou dermatologique telle que définie précédemment est décrit, comprenant le mélange d'un extrait d'agave tel que défini précédemment, et d'un véhicule cosmétiquement et/ou dermatologiquement acceptable.

La composition décrite peut être obtenue par tout procédé approprié connu de l'homme du métier pour la fabrication d'une composition cosmétique. Il peut s'agir, par exemple d'un simple mélange. Il peut s'agir alternativement, par exemple, d'un procédé comprenant une étape d'incorporation d'une phase interne dans une phase externe au moyen d'un émulseur, par exemple d'une turbine de type rotor-stator. Il peut s'agir également par exemple d'un procédé utilisant la Température d'inversion de Phase (TIP), ce procédé étant classiquement utilisé par l'homme de l'art pour obtenir des émulsions huile dans eau dont les gouttelettes dispersées sont particulièrement fines, par exemple avec un diamètre de 0,1 à 1 $\mu$m.

La composition cosmétique ou dermatologique peut comprendre de 0,05 à 5 % en poids d'extraits d'Agave par rapport au poids total de la composition, par exemple de 0,1 à 5%, de 0,1 à 1%, de 0,25 à 1% ou de 0,25 à 0,5%.

La composition cosmétique ou dermatologique décrite peut se trouver sous toute forme appropriée pour une application cosmétique ou dermatologique. Avantageusement, la composition est une composition à usage topique, rincée ou non rincée.

Il peut s'agir par exemple d'une composition sous une forme choisie dans le groupe comprenant une émulsion huile dans eau ou eau dans huile ou un mélange de ces émulsions.

La composition cosmétique ou dermatologique peut par exemple se trouver sous une forme choisie dans le groupe comprenant un gel aqueux ou hydroalcoolique, une crème aqueuse ou hydroalcoolique et une lotion aqueuse ou hydroalcoolique. Ces formulations utilisables pour la mise en oeuvre de la présente invention sont connues dans l'état de la technique par les formulateurs. Dans ces exemples de composition, il suffit d'ajouter des extraits d'Agave tequilana tel que décrits précédemment pour obtenir une composition.

La composition peut être sous une forme choisie parmi un shampooing, un onguent, une crème, une huile, un lait, une pommade, une poudre, une solution, un gel, un sérum, un baume, un beurre, une lotion, une suspension, un savon ou une émulsion, cette forme pouvant être rincée ou non rincée.

L'extrait utilisé dans la présente invention peut être utilisé dans une composition cosmétique ou dermatologique seul ou en combinaison avec d'autres substances ou ingrédients actifs ou inactifs cosmétiquement ou dermatologiquement. Les substances ou ingrédients inactifs sont ceux qui n'agissent pas cosmétiquement ou dermatologiquement. Il s'agit des éléments de la composition permettant notamment d'accompagner l'extrait, de constituer une formulation particulière, de conserver l'extrait actif dans le temps. Il peut s'agir en d'autres termes de tout produit de base pouvant se trouver dans les compositions cosmétiques ou dermatologiques classiques. Par opposition, les substances ou ingrédients actifs sont ceux qui dans l'application cosmétique ou dermatologique visée ont une action esthétique et/ou médicale.

Ainsi, l'extrait d'Agave utilisé dans la présente invention peut être la seule substance ou ingrédient actif d'une composition, ou il peut être associé à d'autres substances ou ingrédients actifs d'une composition cosmétique ou dermatologique.

Un dispositif est décrit, pouvant se présenter sous une forme choisie parmi un pot, un flacon, un flacon-pompe, un masque, un tube, un sachet soudé, un spray, ledit dispositif comprenant un extrait ou une composition décrite.

Un procédé de soin non thérapeutique, cosmétique ou dermatologique est décrit, permettant notamment d'apporter un effet d'amélioration de la croissance du cheveu. Le procédé peut comprendre une application sur le cheveu et/ou le cuir chevelu d'une composition cosmétique décrite.

Une utilisation d'une composition cosmétique ou dermatologique telle que définie précédemment est décrite, pour son utilisation dans un produit dermatologique destiné à l'amélioration de la croissance du cheveu d'un sujet.

Dans le cadre des procédés cosmétiques, ou de l'utilisation décrits, l'utilisation s'entend d'une utilisation non-thérapeutique, par exemple pour le traitement des peaux et cheveux sains, c'est-à-dire notamment des cuirs chevelus ne présentant pas un état pathologique ou, si le cuir chevelu présente un état pathologique, pour une utilisation strictement esthétique, à l'exclusion de toute utilisation thérapeutique. En effet, l'utilisation cosmétique et esthétique décrite n'est pas associée à un effet thérapeutique inévitable.

Ainsi, toute utilisation cosmétique et tout procédé cosmétique décrits sont respectivement des utilisations cosmétiques non-thérapeutiques et des procédés cosmétiques non-thérapeutiques, ne visant pas à traiter une pathologie.

D'autres avantages pourront encore apparaître à l'homme du métier à la lecture des exemples ci-dessous, donnés à titre illustratif.

## EXEMPLES

### Exemple 1 : Préparation des extraits d'Agave

La matière première utilisée pour la production des fructooligosaccharides de degré de polymérisation inférieur ou égal à 10 (produit commercial Metlos®) et des inulines de degré de polymérisation supérieur à 10 (produit commercial Metlin®) est l'Agave bleu biologique récoltée dans les hauts plateaux de l'État de Jalisco. Après 5 à 7 ans de croissance soigneusement contrôlée, l'Agave a la quantité maximale de fructanes, et est donc prête à être transformée.

L'agave est reçue fraîche et lavée à l'arrivée pour éliminer autant de matières étrangères que possible. L'agave est ensuite broyée en trois étapes pour passer de 40 à 100 kg de noyau d'agave à une bagasse pulpeuse, prête à être transformée pour en extraire les glucides.

La liqueur brute obtenue à partir de l'extraction est ensuite clarifiée pour éliminer toute couleur et impuretés restantes. Ensuite, le jus clarifié est purifié en deux fractions: Metlos® et Metlin®. Chaque produit est ensuite amené à une dernière étape de purification afin d'éliminer tous les minéraux pour obtenir une pureté élevée (> 99% de glucides).

### Exemple 2 : Effet d'extraits d'Agave obtenus dans l'Exemple 1 sur le renouvellement des kératinocytes épidermiques

Les études ont été réalisées sur des kératinocytes épidermiques humains normaux, obtenus à partir de plasties abdominales, ensemencés en monocouche dans les conditions de culture adéquates, à savoir dans un milieu KSFM appauvri (« Keratinocyte Sérum Free Medium»), à une température de 37°C, sous atmosphère à 5% de $CO_2$ et saturée en humidité. Les cellules ont été traitées avec deux types d'extraits d'Agave (Metlin® ou Metlos® à $10^{-1}$%, $10^{-2}$ % ou $10^{-3}$%).

Après 3 ou 7 jours de traitement (test en triplicata et reproduit 2 fois), la viabilité cellulaire a été évaluée par quantification de l'activité métabolique des déshydrogénases mitochondriales, par mesure d'hydrolyse du MTT (« MethylThiazoleTetrazolium »).

Les données du tableau I ci-dessous concernent le pourcentage de viabilité des kératinocytes, ayant subi différents traitements.

La référence positive est le milieu KSFM complet (avec Bovine Pituitary Extract).

Tableau I : Pourcentage de viabilité des kératinocytes

| Durée Traitement (jours) | Milieu base | KSFM complet | Inuline Metlin® | | | Inuline Metlos® | | |
|---|---|---|---|---|---|---|---|---|
| | | | $10^{-1}$% | $10^{-2}$% | $10^{-3}$% | $10^{-1}$% | $10^{-2}$% | $10^{-3}$% |
| 3 | 100 | 159 | 133 | 131 | 141 | 136 | 132 | 132 |
| 7 | 100 | 148 | 120 | 123 | 118 | 85 | 107 | 113 |

Cet exemple montre que l'adjonction de l'un ou l'autre des extraits d'Agave dans le milieu de culture permet aux kératinocytes de proliférer d'avantage jusqu'à 41 % à $10^{-3}$% pour l'inuline Metlin® et 36% à $10^{-1}$% pour l'inuline Metlos®. Le renouvellement des kératinocytes étant impliqué dans le lissage et l'homogénéité de surface du cheveu et du cuir chevelu, ce test valide l'intérêt de ces extraits.

### Exemple 3 : Effet d'extraits d'Agave obtenus dans l'Exemple 1 sur la formation de microtubules par les cellules endothéliales.

Dans cette expérience les cellules sont des cellules endothéliales de veines ombilicales humaines (HUVEC) cultivée dans du milieu M200 (Invitrogen M200500) sur du matrigel (BD Biosciences 354234) pendant 6 heures. Les cellules sont soit dans le milieu (témoin milieu), soit traitées par la suramine (témoin négatif), soit traitées par le VEGF (témoin positif), soit traitées par les inulines Metlin® ou Metlos® (à 0,1%). Après 6 heures d'incubation, les pseudo-tubes formés sont observés en microcopie et des images sont prises aléatoirement pour ensuite être quantifiées (surface totale du réseau) par analyse d'images.

|  | Témoin milieu | T+ (VEGF) | T-(Suramine) | Inuline Metlin® | Inuline Metlos® |
|---|---|---|---|---|---|
| Concentrations | - | 50 ng/ml | 20 μM | 0,1% | 0,1% |
| Nombre de ramifications | 13 | 18 | 0 | 28 | 27 |
| Résultats rapportés en % | 100 | 140 | 0 | 220 | 216 |

Le traitement avec les inulines Metlin® ou Metlos® permet de stimuler la formation de pseudo tubes de 220% à 0,1% pour Metlin® et de 216% à 0,1% pour Metlos®. Ces résultats valident donc l'utilisation de l'extrait dans un soin cosmétique visant à stimuler la croissance du cheveu.

**Exemple 4 : Effet d'extraits d'Agave obtenu dans l'Exemple 1 sur la migration des cellules endothéliales.**

L'étude a été réalisée sur des PEC (progéniteurs endothéliaux circulants) issus de sang de cordon humain, cultivées dans le milieu EGM-2 (Endothelial Growth medium-2).
Avant le traitement les cellules sont sevrées dans du milieu EBM-2 0,2% de sérum de veau foetal (SVF). Les cellules sont ensuite ensemencées dans un insert contenant du milieu EGM-2 avec 0,2% SVF, baignant dans un puits contenant le traitement : soit le milieu EGM-2 0,2% SVF (témoin négatif), soit le milieu EGM-2 0,2% SVF plus le VEGF à 10 ng/ml (témoin positif), soit le milieu EGM-2 0,2% SVF plus les inulines Metlin® ou Metlos® à 0,5% ou 1%. Après 5 heures d'incubation, les cellules à l'intérieur des inserts sont éliminées et celles ayant migré de l'autre côté de la membrane de l'insert sont fixées au paraformaldéhyde 4% pendant 10 min. Les cellules ayant migré sont ensuite comptées.

|  | T-(milieu seul) | T+ (milieu + VEGF) | Inuline Metlin® 0,5% | | Inuline Metlin® 1% | | Inuline Metlos® 0,5% | | Inuline Metlos® 1% | |
|---|---|---|---|---|---|---|---|---|---|---|
| VEGF (10 ng/ml) | - | + | - | + | - | + | - | + | - | + |
| Nombre de cellules | 231 | 417 | 356 | 609 | 404 | 672 | 271 | 489 | 328 | 504 |
| Résultats rapportés au T- | 100% | 181% | 155% | 264% | 175% | 291% | 178% | 243% | 185% | 280% |

Le traitement avec les inulines Metlin® ou Metlos® permet de stimuler la migration des progéniteurs endothéliaux humains de 75% à 1 % pour Metlin® et de 85 % à 1 % pour Metlos® en condition basale (non stimulée par le VEGF). En condition stimulée, Le traitement avec les inulines Metlin® ou Metlos® permet de stimuler la migration des progéniteurs endothéliaux humains de 191% à 1 % pour Metlin® et de 180 % à 1% pour Metlos®.
Ces résultats valident donc l'utilisation de l'extrait dans un soin cosmétique visant à stimuler la vascularisation du follicule pileux et ainsi la croissance du cheveu.

**Exemple 5 : Évaluation de l'effet des inulines par des mesures tensiométriques de la surface des cheveux**

L'objectif de cette étude est d'évaluer l'effet de Metlin® et de Metlos®, notés respectivement actif A et actif B par des mesures de l'angle de contact de l'eau sur la surface des cheveux.
Les cheveux sains sont naturellement protégés par une couche de surface lipidique avec des propriétés hydrophobes.
Or, l'hydrophobicité des cheveux diminue après des dommages chimiques engendrés par exemple par la décoloration. Le but de la présente étude est de mesurer l'augmentation de l'hydrophobicité sur la surface des cheveux après immersion dans une solution d'actifs nourrissante à base de fructanes (inulines et/ou fructo-oligosaccharides), dues à l'amélioration de l'hydrophobicité sur les cheveux.

Principe de l'étude

La mesure est basée sur la méthode de Wilhelmy. Il s'agit d'une méthode actuellement utilisée pour mesurer l'angle de contact d'un liquide sur une seule fibre. La fibre (le cheveu) ne doit pas être perméable au liquide utilisé (l'eau) et la tension superficielle de ce liquide doit être connue.

Le principe consiste en l'utilisation d'une balance de haute précision afin de mesurer la force nécessaire lors de l'immersion des cheveux dans l'eau. L'angle de contact est calculé à partir de l'équation de Wilhelmy:

$$\gamma = \frac{F}{L \times \cos \theta}$$

Avec :

γ : Tension de surface de l'eau : 72,8 mN/m
F : force
L : longueur à l'état mouillé (mesuré précédemment)
θ : angle de contact

Comme indiqué ci-dessus, la mesure de l'angle de contact nécessite une détermination préalable de la longueur mouillée de l'échantillon. La mesure de la longueur mouillée s'effectue à l'aide d'un solvant à faible tension superficielle, l'hexane dont la tension superficielle est de 18,4 mN/m, pour laquelle on considère que l'angle de contact est nul.

Quant à la mesure de l'angle de contact, l'échantillon est graduellement immergé dans l'hexane, la force d'immersion est mesurée. La force F exercée juste en contact avec le solvant est déterminée par régression linéaire, la et par calcul, et la longueur mouillée avec l'équation de Wilhelmy.

Données du test

| Mèche | Naturelle - traitée |
|---|---|
| T Nat | Natural standard (bruns européens, fournisseur : Quimdis) |
| D2 | D2 - Standard |
| D2 + Traitement | D2 + 1 application par immersion rincée ou non dans la solution d'actif |

Origine des cheveux: Cheveux bruns européens.

Les cheveux dits « D2 » ou « D2 standard » sont des cheveux bruns européens (fournisseur : Quimdis) ayant subit 2 cycles de décolorations. La décoloration est l'éclaircissement de la nuance naturelle des cheveux par la modification chimique (procédés d'oxydation alcaline) des caractéristiques des pigments de mélanine. Le processus, très progressif, de décoloration du pigment (solubilisation par dépolymérisation de la mélanine) est précédé de l'attaque de la kératine et des polypeptides stabilisant le pigment mélanique par le traitement décolorant (rupture des liaisons disulfures, amides, salines et hydrogènes). On note D2 les cheveux ayant subi une décoloration répétée deux fois. Nous réalisons ce processus en laboratoire par immersion des mèches dans une solution à base de peroxodisulfate de sodium et de peroxyde d'hydrogène.

Les inulines ou oligo-fructosaccharides utilisées sont Metlin® ou Metlos® (NEKUTLI) et leurs mélanges dont les ratios Metlin®/ Metlos® sont les suivants : 100/0, 75/25, 50/50, 25/75 et 0/100.

Les concentrations des inulines et/ou oligo-fructosaccharides dans les solutions aqueuses d'actifs appliquées sont de 0,25%, 0,5% et 1%. Ce pourcentage correspond au produit total dans le produit, c'est-à-dire soit l'inuline lorsque le produit ne contient pas de oligo-fructosaccharides, soit les oligo-fructosaccharides lorsque le produit ne contient pas d'inuline, soit le mélange des deux lorsque le produit comporte de l'inuline et des oligo-fructosaccharides.

Dans certains cas, les cheveux ont été rincés après l'application des inulines et/ou oligo-fructosaccharides après 5 minutes d'immersion dans la solution aqueuse d'actifs, tandis que dans les autres cas, les cheveux n'ont pas été rincés.

Le tensiomètre utilisé est le tensiomètre Krüss K100SF.

Protocole d'échantillonnage

10 cheveux par mèche ont été mesurés.

Pour chaque cheveu, le protocole suivant est utilisé:

- Le cheveu est coupé de la mèche à l'aide de ciseaux propres sans autre manipulation et manipulés avec une pince propre.
- Il est fixé sur sa partie supérieure sur une feuille de papier au moyen d'un adhésif double face.
- Pour chaque mesure, un échantillon d'une longueur de 15 mm est coupé à l'aide de ciseaux propres.
- Les 3 premiers échantillons sont utilisés pour mesurer la longueur mouillée.
- Les 3 échantillons suivants sont utilisés pour mesurer l'angle de contact.
- L'échantillon est fixé sur la surface collante du porte-échantillon, sur une longueur d'environ 5 mm (fixation directe sur le porte-échantillon sans autre manipulation). L'échantillon doit être aussi droit que possible, pour venir perpendiculairement au contact du liquide, de manière à ne pas fausser la mesure.

La mesure est effectuée à 5 mm du fond de l'échantillon.

Conditions de mesure

Mesure de longueur mouillée

| paramètres: | valeur |
| --- | --- |
| Vitesse de détection | 6 mm/min |
| Sensibilité de détection | $5.10^{-5}$ g |
| Mesure de la vitesse | 3 mm/min |
| Différence de position | 0,2 mm |
| Profondeur d'immersion | 5 mm |

Contrôle de l'eau

Les mesures sont effectuées avec de l'eau du robinet qui est systématiquement contrôlée tous les jours lors des mesures.

Résultats:

| température | pH | Surface tension (mN/m) |
| --- | --- | --- |
| 23 °C | 7.4$\pm$1 | 72,7 mN/m (s=0.1) |

Résultats des tests d'hydrophobicité:

Les résultats sont donnés au moyen d'un calcul d'un test W de Mann-Whitney pour comparer les médianes des deux échantillons comparés, les échantillons étant indépendants. Les échantillons comparés sont les cheveux abimés par décoloration (D2) et les cheveux abimés par décoloration (D2) et traités avec la solution aqueuse d'actifs.

RESULTATS DES TESTS HYDROPHOBICITE 1% (Actif A : Inuline-Metlin®, Actif B : Metlos®)

| références | Mèche 1 : cheveux naturels Angle de contact (°) | | Mèche 2 : cheveux abîmés (décolorés) Angle de contact (°) | |
|---|---|---|---|---|
| | reg | moy | reg | moy |
| moy | 102,4 | 101,5 | 78,2 | 77,8 |
| Ecart type | 2,9 | 2,4 | 11,8 | 11,0 |
| Coef variation % | 2,8 | 2,3 | 15,1 | 14,1 |

| Ratio A/B | 100/0 | | 75/25 | | 50/50 | | 25/75 | | 0/100 | |
|---|---|---|---|---|---|---|---|---|---|---|
| Non rincé | Mèche 3 | | Mèche 4 | | Mèche 5 | | Mèche 6 | | Mèche 7 | |
| | reg | moy | reg | moy | reg | moy | reg | moy | reg | moy |
| moy | 91,8 | 90,2 | 83,7 | 83,7 | 87,1 | 85,2 | 84,5 | 82,6 | 84,4 | 82,7 |
| Ecart type | 7,4 | 7,8 | 13,5 | 12,3 | 13,2 | 13,6 | 11,5 | 11,5 | 15,2 | 15,7 |
| Coef variatio n % | 8,1 | 8,7 | 16,1 | 14,7 | 15,2 | 15,9 | 13,6 | 13,9 | 18,1 | 19,0 |

| Ratio A/B | 100/0 | | 75/25 | | 50/50 | | 25/75 | | 0/100 | |
|---|---|---|---|---|---|---|---|---|---|---|
| Rincé | Mèche 8 | | Mèche 9 | | Mèche 10 | | Mèche 11 | | Mèche 12 | |
| | reg | moy | reg | moy | reg | moy | reg | moy | reg | moy |
| moy | 90,8 | 90,5 | 87,7 | 85,7 | 87,7 | 84,7 | 83,4 | 82,1 | 87,4 | 85,9 |
| Ecart type | 11,6 | 11,4 | 7,0 | 7,4 | 10,6 | 11,4 | 9,5 | 8,8 | 8,3 | 8,7 |
| Coef variation % | 12,7 | 12,6 | 8,0 | 8,6 | 12,1 | 13,4 | 11,4 | 10,8 | 9,5 | 10,1 |

**Une différence significative par rapport au D2 est constatée.**

RESULTATS DES TESTS HYDROPHOBICITE 0.5% et 0.25% (Actif A : Inuline-Metlin®, Actif B : Metlos®)

| références | Mèche 1 : cheveux naturels Angle de contact (°) | | Mèche 2 : cheveux abîmés (décolorés) Angle de contact (°) | |
|---|---|---|---|---|
| | reg | moy | reg | moy |
| moy | 105,6 | 104,2 | 68,0 | 67,2 |
| Ecart type | 3,4 | 3,5 | 15,3 | 16,6 |
| Coef variation % | 3,2 | 3,4 | 22,5 | 24,6 |

**Une différence significative par rapport au D2 est constatée.**

| Ratio A/B | 50/50 - 0,5% Angle de contact (°) | | 50/50 - 25% Angle de contact (°) | |
|---|---|---|---|---|
| | reg | moy | reg | moy |
| moy | 77,6 | 76,8 | 69,5 | 68,0 |
| Ecart type | 13,2 | 12,2 | 7,1 | 6,5 |

(suite)

| Ratio A/B | 50/50 - 0,5% Angle de contact (°) | | 50/50 - 25% Angle de contact (°) | |
|---|---|---|---|---|
| | reg | moy | reg | moy |
| Coef variation % | 17,0 | 15,8 | 10,2 | 9,6 |
| P =0.00355455**s | | | P =0.0929457****ns | |

**Une différence significative par rapport au D2 est constatée pour le ratio 50/50 à 0.5%.**

On constate donc une différence significative du ratio 50/50 à 0,5% rincé par rapport au témoin décoloré, et une différence non significative par rapport au 50/50 1% Rincé.

RESULTATS DES TESTS HYDROPHOBICITE Tous les ratios rincés et non rincés à 1% +Essais 50/50 rincé à 0.5% et 0.25%

On constate un apport significatif d'hydrophobicité pour les traitements :

- Rincé à 1% : tous les ratios (moindre pour le 25/75)
- Rincé à 0,5% pour le ratio 50/50
- Non rincé à 1% tous les ratios (moindre pour les 75/25 et 0/100)

**Exemple 6 : Formulation d'un extrait d'Agave dans un sérum concentré pour soin végétal capillaire**

| Composants (nom INCI) | Pourcentage (% en poids) |
|---|---|
| AQUA | 54,575 |
| GELLAN GUM | 0,150 |
| SODIUM CHLORIDE | 0,075 |
| SODIUM BENZOATE | 0,500 |
| POTASSIUM SORBATE | 0,200 |
| SALICYLIC ACID | 0,150 |
| BETAINE | 2,000 |
| LACTIC ACID & AQUA | 0,150 |
| PANTHENOL | 0,200 |
| ALOE BARBADENSIS LEAF JUICE | 40,000 |
| FRUCTOOLIGOSACCHARIDES | 1,000 |
| INULIN | 1,000 |

**Exemple 7 : Formulation d'un extrait d'Agave dans un shampooing**

| Composants (nom INCI) | Pourcentage (% en poids) |
|---|---|
| AQUA | 76,660 |
| GUAR HYDROXYPROPYLTRIMONIUM CHLORIDE | 0,150 |
| CITRIC ACID | 0,090 |
| SODIUM BENZOATE | 0,500 |

(suite)

| Composants (nom INCI) | Pourcentage (% en poids) |
|---|---|
| ZINC GLUCONATE | 0,500 |
| SALICYLIC ACID | 0,050 |
| FRUCTOOLIGOSACCHARIDES | 0,125 |
| INULIN | 0,125 |
| COCO-GLUCOSIDE & GLYCERYL OLEATE & AQUA | 3,000 |
| DECYL GLUCOSIDE & AQUA | 3,000 |
| AMMONIUM LAURYL SULFATE & AQUA | 10,400 |
| COCAMIDOPROPYL BETAINE & AQUA | 4,000 |
| PARFUM | 0,400 |
| URTICA DIOICA EXTRACT & AQUA & GLYCERIN | 1,000 |

**Exemple 8 : Formulation d'un extrait d'Agave dans un soin nutrition pour les cheveux sous forme d'après-shampooing**

| Composants (nom INCI) | Pourcentage (% en poids) |
|---|---|
| AQUA | 87,967 |
| INULIN | 0,250 |
| FRUCTOOLIGOSACCHARIDES | 0,250 |
| PANTHENOL | 0,100 |
| SODIUM BENZOATE | 0,350 |
| BEHENTRIMONIUM CHLORIDE & ISOPROPYL ALCOHOL | 3,000 |
| STEARYL ALCOHOL | 3,500 |
| CETYL ALCOHOL | 2,000 |
| PERSEA GRATISSIMA OIL | 1,000 |
| COCOS NUCIFERA OIL | 1,000 |
| PARFUM | 0,400 |
| OLEA EUROPAEA FRUIT OIL & BETA-CAROTENE | 0,003 |
| CITRIC ACID | 0,180 |

**REFERENCES BIBLIOGRAPHIQUES**

1. Maria de la Soledad ALONSO GUTIÉRREZ, thèse, « Valorisation de la bagasse de l'agave tequilana W. cv azul : caractérisation, étude de la digestibilité et de la fermentation des sucres », 2005.
2. López, M.G. ; Mancilla, N.A. ; Mendoza-Díaz, G. (2003). : Molecular structures of fructans from Agave tequilana Weber var. azul. J. Agric. Chem. 51, 7835-7840.

**Revendications**

1. Utilisation cosmétique non thérapeutique d'un extrait d'Agave *tequilana* pour une amélioration de la croissance du cheveu, dans laquelle ledit extrait d'agave comprend des fructo-oligosaccharides ayant un degré de polymérisation inférieur ou égal à 10 et/ou des inulines ayant un degré de polymérisation compris de 10 à 60.

2. Utilisation cosmétique selon la revendication 1, dans laquelle ledit extrait d'Agave stimule le renouvellement des kératinocytes.

3. Utilisation cosmétique selon l'une quelconque des revendications précédentes, dans laquelle ledit extrait d'Agave stimule la formation des microtubules et/ou la migration des cellules endothéliales.

4. Utilisation cosmétique selon l'une quelconque des revendications précédentes, dans laquelle lesdits fructo-oligosaccharides ont un degré de polymérisation compris de 3 à 10.

5. Utilisation cosmétique selon l'une quelconque des revendications précédentes, dans laquelle le ratio fructo-oligosaccharides sur inulines est compris de 100/0 à 0/100.

6. Utilisation cosmétique selon la revendication 5, dans laquelle le ratio fructo-oligosaccharides sur inulines est de 50/50.

**Patentansprüche**

1. Nicht-therapeutische kosmetische Verwendung eines Extrakts aus *Agave tequilana* zur Verbesserung des Haarwachstums, wobei der Agavenextrakt Fructo-Oligosaccharide mit einem Polymerisationsgrad von 10 oder weniger und/oder Inuline mit einem Polymerisationsgrad von 10 bis 60 umfasst.

2. Kosmetische Verwendung nach Anspruch 1, wobei der Agavenextrakt die Erneuerung von Keratinozyten stimuliert.

3. Kosmetische Verwendung nach einem der vorhergehenden Ansprüche, wobei der Agavenextrakt die Bildung von Mikrotubuli und/oder die Migration von Endothelzellen stimuliert.

4. Kosmetische Verwendung nach einem der vorhergehenden Ansprüche, wobei die Fructo-Oligosaccharide einen Polymerisationsgrad von 3 bis 10 aufweisen.

5. Kosmetische Verwendung nach einem der vorhergehenden Ansprüche, wobei das Verhältnis von Fructo-Oligosacchariden zu Inulinen im Bereich von 100/0 bis 0/100 liegt.

6. Kosmetische Verwendung nach Anspruch 5, wobei das Verhältnis von Fructo-Oligosacchariden zu Inulinen 50/50 beträgt.

**Claims**

1. Non-therapeutic cosmetic use of an extract of *Agave tequilana* for improving hair growth, in which the said agave extract comprises fructo-oligosaccharides having a degree of polymerisation of less than or equal to 10 and/or inulins having a degree of polymerisation of between 10 and 60.

2. Cosmetic use according to claim 1, in which said Agave extract stimulates the renewal of keratinocytes.

3. Cosmetic use according to any one of the preceding claims, in which the said Agave extract stimulates the formation of microtubules and/or the migration of endothelial cells.

4. Cosmetic use according to any one of the preceding claims, in which said fructo-oligosaccharides have a degree of polymerisation of between 3 and 10.

5. Cosmetic use according to any one of the preceding claims, in which the ratio of fructo-oligosaccharides to inulins is between 100/0 and 0/100.

6. Cosmetic use according to claim 5, in which the ratio of fructo-oligosaccharides to inulins is 50/50.

## RÉFÉRENCES CITÉES DANS LA DESCRIPTION

**Documents brevets cités dans la description**

- CN 106420461
- JP 2000136142 A
- BR 102012012704, De Campos Catarina Ledi
- ES 2009151, Llaurado Grau José Maria ; Lopez Valverde Juan Antonio
- FR 2551972, Boudiz Moussaad
- EP 1541117 A

**Littérature non-brevet citée dans la description**

- **MARIA DE LA SOLEDAD ALONSO GUTIÉRREZ.** *Valorisation de la bagasse de l'Agave tequilana W. cv azul : caractérisation, étude de la digestibilité et de la fermentation des sucres,* 2005
- **LÓPEZ, M.G. ; MANCILLA, N.A. ; MENDOZA-DÍAZ, G.** Molecular structures of fructans from Agave tequilana Vlleber var. azul. *J. Agric. Chem.,* 2003, vol. 51, 7835-7840
- **MARIA DE LA SOLEDAD ALONSO GUTIÉRREZ.** *Valorisation de la bagasse de l'agave tequilana W. cv azul : caractérisation, étude de la digestibilité et de la fermentation des sucres,* 2005
- **LÓPEZ, M.G. ; MANCILLA, N.A. ; ENDOZA-DÍAZ, G.** Molecular structures of fructans from Agave tequilana Weber var. azul. *J. Agric. Chem.,* 2003, vol. 51, 7835-7840